Europäisches Patentamt

European Patent Office

Office européen des brevets

(19)

(11) Numéro de publication: **0 377 744**
**A1**

(12)

# DEMANDE DE BREVET EUROPEEN PUBLI E EN APPLICATION DE L'article 158, paragraphe 3 de la CBE

(21) Numéro de dépôt: 89901151.4

(22) Date de dépôt: 26.05.88

(86) Numéro de dépôt internationale :
PCT/SU88/00131

(87) Numéro de publication internationale :
WO 89/11254 (30.11.89 89/28)

(51) Int. Cl.⁵: **A61B 17/60**

(43) Date de publication de la demande:
**18.07.90 Bulletin 90/29**

(84) Etats contractants désignés:
**AT BE CH DE FR GB IT LI SE**

(71) Demandeur: **VSESOJUZNY KURGANSKY
NAUCHNY TSENTR "VOSSTANOVITELNAYA
TRAVMATOLOGIA I ORTOPEDIA"**
**ul. M.Ulyanovoi, 6**
**Kurgan, 640005(SU)**

(72) Inventeur: **ILIZAROV, Gavriil Abramovich**
**ul. Klimova, 41-38**
**Kurgan, 640020(SU)**

(74) Mandataire: **Durand, Yves Armand Louis et al**
**CABINET WEINSTEIN 20, Avenue de
Friedland**
**F-75008 Paris(FR)**

(54) **APPAREIL DE COMPRESSION-EXTENSION POUR OSTEOSYNTHESE.**

(57) Appareil de compression-extension pour ostéosynthèse du type comportant au moins deux centres d'appui (1) qui comprennent au moins deux broches (3) fixées dans des porte-broches (2) réalisés en forme de boulon et comportant des plaques avec des rainures ouvertes pour l'emplacement des broches (3) ainsi que des joints, les porte-broches étant installés sur une tige filetée (6) avec possibilité de déplacement le long de son axe, lequel boulon du porte-broche (2) comporte un orifice axial au travers duquel passe la tige filetée principale (6), les plaques et les joints étant réalisés en forme de rondelles disposées sur le boulon, les rainures ouvertes pour l'installation des broches (3) étant quant à elles réalisées sur les faces en bout des plaques et passent le long de la corde de leur circonférence à distance de l'orifice et à l'intérieur de la rondelle, les broches (3) étant repliées et fixées dans le porte-broche (2) de façon à se croiser.

FIG.1

"Appareil de compression-extension pour ostéosynthèse".

## Domaine de l'invention

L'invention se rapporte à la médecine et plus particulièrement au domaine de l'orthopédie et de la traumatologie, et a pour objet des appareils de compression-extension pour ostéosynthèse.

## Etat de la technique

On connaît un appareil externe pour la fixation axiale orthopédique (FR-A-255938). Ledit appareil comporte une tige métallique en forme de parallélépipède qui porte deux pinces à broche. Les mors des pinces à broche se fixent au moyen de vis. L'une de ces pinces est installée de façon à pouvoir se déplacer longitudinalement par le biais d'une broche filetée.

L'appareil susmentionné permet de fixer les os tubulaires et de réaliser le déplacement longitudinal des fragments osseux. Cependant lors de la disposition parallèle des broches fixées en console, il se produit un glissement du dernier de ces os à la suite de l'application d'une charge. D'autre part, les rainures pour les broches formées dans les pinces sont réalisées avec un écart déterminé l'une de l'autre et rendent nécessaire la disposition des broches dans un site défini avec précision, ce qui n'est pas toujours possible pour le segment donné de l'os.

Pour le traitement de lésions de l'os, on utilise un appareil orthopédique destiné à la fixation axiale extérieure de l'os ou des fragments osseux de la fracture avec un large spectre de régulation (GB-A-2168255).

Ledit appareil comporte un corps central, composé de trois parties ou plus, qui ont la possibilité de se déplacer l'une par rapport à l'autre grâce à un glissement télescopique sans possibilité de rotation relative de ces parties. Les parties du corps sont munies de moyens de blocage qui peuvent être actionnés sélectivement pour chaque paire desdites parties.

Par ailleurs, ledit appareil connu est muni d'un vérin de commande qui a au moins trois éléments qui peuvent se visser l'un dans l'autre et se déplacer le long d'un axe commun suite à la rotation de l'un desdits éléments. Les éléments se situent sur le bout du vérin et sont munis de goupilles qui peuvent entrer dans des cavités excentriques du corps cylindrique.

L'appareil connu susmentionné permet le réglage de la position des éléments munis de goupilles par rapport au corps central ainsi que l'un par rapport à l'autre. Néanmoins, les goupilles sont disposées dans les orifices des éléments à un écart déterminé l'un de l'autre, ce qui rend complexe l'utilisation de l'appareil à cause de l'absence de choix libre du site d'installation des broches dans l'os. L'appareil orthopédique destiné à la fixation extérieure axiale ne permet pas la régulation de l'angle de disposition des broches dans l'élément et par ailleurs ne permet de faire passer les broches que dans le sens dos-paume ce qui entraîne la détérioration des flexeurs de la main.

Pour le traitement des lésions des articulations de la main on utilise un fixateur extérieur dynamométrique (GB-A-2146533) possédant deux appuis longitudinaux solidaires de broches de fixation qui se placent dans chacun de ces appuis. Les deux appuis se réunissent au moyen d'une charnière universelle qui comporte des éléments de réglage du degré de déplacement de l'appui distal par rapport à l'appui le plus proche. L'appui le plus proche comporte plusieurs orifices pour l'introduction de broches de fixation et d'éléments filetés de blocage correspondants. L'appui distal est muni d'un plongeur de réglage de sa longueur. On utilise une vis d'ajustage qui se déplace alternativement dans la rainure de l'appui distal pour régler le degré d'ouverture vers l'extérieur du plongeur. Le fixateur externe dynamométrique susmentionné permet de fixer l'articulation de deux os adjacents le long de la face latérale du carpe.

Le fixateur connu susmentionné offre la possibilité de régler le degré de déplacement de l'appui distal par rapport à l'appui le plus proche et d'ajuster la valeur de ce déplacement. En outre, les broches de l'appui distal ont la possibilité de se déplacer par rapport aux broches de l'appui le plus proche. Cependant, les orifices pour les broches formées dans les appuis du fixateur susmentionné sont réalisés sur un écart fixé l'un par rapport à l'autre. On ne peut donc pas installer et fixer les broches suivant différents angles par rapport à l'axe longitudinal des appuis du fixateur. Les broches se situent dans un même plan qui est parallèle à l'axe longitudinal de l'os. D'autre part il s'avère assez difficile de poser le fixateur externe dynamométrique connu sur des phalanges adjacentes et de le poser simultanément sur des os adjacents du métacarpe et sur les phalanges.

Avant la mise en place des broches de fixation à l'aide des bouts filetés il faut exécuter un canal dans l'os, puis y engager le bout fileté de la broche, ce qui produit une traumatisation des parties molles et du tissu de l'os. Au cours du traitement il peut se produire une résorption de l'os qui entraîne le glissement des broches de fixation de l'os avec leurs bouts filetés.

Dans le traitement des lésions des os, on utilise des minifixateurs externes (Klinki für orthopadische Chirurgie der Universität Bern, Schweiz, Roland P. Jakob "Der Kleine Fixateur externe"). Ces fixateurs comportent des tiges reliées entre elles et des broches avec des extrémités filetées installées dans des pinces à broches. Les broches sont installées et fixées suivant un angle compris entre 40 et 60°.

Le dispositif susmentionné permet de réaliser une réduction dosée, puis de fixer les os lésés. Les broches se fixent dans un plan parallèle à l'axe longitudinal de la tige qui porte les pinces à broches, chacune de ces dernières ne portant qu'une seule broche ce qui entraîne une augmentation de l'encombrement longitudinal du fixateur. Par ailleurs, il peut se produire au cours du traitement une résorption de l'os entraînant l'ébranlement des broches.

Il existe un appareil plus perfectionné pour l'allongement des os tubulaires courts (SU-A-1161100) qui permet une extension de l'os. Cet appareil comprend une tige filetée sur laquelle se placent les pinces à broches pouvant se déplacer l'une par rapport à l'autre. Chaque pince à broche est réalisée en forme de plaques d'appui et de serrage avec des rainures, des boulons et des rondelles venant se placer entre lesdites plaques. La plaque d'appui est installée sur la tige filetée, et les boulons sont installés dans les orifices sans filet des rondelles et de la plaque d'appui, ainsi que dans les orifices filetés de la plaque de serrage.

Cependant l'appareil susmentionné ne permet pas de faire varier l'angle de passage des broches car les rainures des plaques d'appui et de serrage ne peuvent être placées à différentes positions angulaires par rapport au plan qui passe par les axes longitudinaux des tiges filetées, ce qui rend impossible la correction de la position des broches au cours du traitement lorsque survient une résorption de l'os aux sites de contact avec les broches, ainsi que la réduction et l'utilisation dudit appareil pour la guérison des fractures. Les essais cliniques d'utilisation dudit appareil ont révélé les possibilités limitées pour l'ostéosynthèse des os tubulaires courts adjacents à cause de son grand encombrement.

Exposé de l'invention

Le problème posé dans la présente invention consiste à mettre au point un appareil de compression-extension dont la construction assurerait le positionnement des broches des sites optimum de l'os, ainsi que la correction de la position des broches au cours de l'ostéosynthèse et du traitement ultérieur et de plus la possibilité d'élargir ses capacités fonctionnelles grâce à la réduction de ses dimensions et de son poids.

Ce problème est résolu par le fait que l'appareil de compression-extension pour ostéosynthèse comporte au moins deux centres d'appui qui comprennent au moins deux broches dont les axes de l'une des branches se croisent dans l'os et les autres se fixent dans des porte-broches réalisés en forme de boulons supportant un ensemble de plaques avec des rainures ouvertes pour l'installation des broches et un ensemble de joints, les plaques et les joints étant installés sur la tige filetée avec la possibilité de se déplacer le long de son axe et de se fixer au moyen d'écrous. Suivant l'invention, le boulon du porte-broche a un orifice

- 6 -

axial par lequel passe la tige filetée principale, les plaques et les joints sont réalisés en forme de rondelles installées sur le boulon avec possibilité de rotation autour de son axe et se fixent par un écrou de serrage. Les rainures ouvertes pour l'emplacement des broches sont exécutées sur les surfaces en bout des plaques et passent le long de la corde de leur circonférence à distance de l'orifice de la rondelle et les écrous sont installés sur la tige filetée principale et se disposent des deux côtés des extrémités du boulon, les broches étant courbées et fixées dans le porte-broche par croisement des axes.

Le porte-broche du centre d'appui est muni d'un dispositif de fixation, tel que par exemple une vis d'arrêt installée dans un orifice fileté réalisé dans la tête du boulon du porte-broche, perpendiculairement à l'axe de son orifice, et d'un méplat exécuté le long de la tige filetée principale et avec lequel l'about de la vis d'arrêt coopère.

L'utilisation de la vis d'arrêt installée dans l'orifice fileté réalisé dans la tête du boulon du porte-broche du centre d'appui et son interaction avec le méplat le long de la tige filetée principale empêche la rotation du centre d'appui au cours de son déplacement dosé le long de l'axe longitudinal de la tige filetée principale.

Il est possible de réaliser sur la face en bout de la tête du boulon du porte-broche une rainure pour l'installation de la broche située du côté du filet.

L'exécution de cette rainure permet de meilleures conditions de positionnement de la broche et permet de plus d'installer et de fixer la broche dans le centre d'appui avec une quantité réduite de plaques et de joints.

Il est avantageux de réaliser chaque plaque du porte-broche avec une rainure ouverte en forme de rondelle de façon qu'un méplat dont le plan est incliné par rapport à la face de bout de la plaque et qui est parallèle à l'axe longitudinal de la rainure, le méplat et la rainure étant exécutés du même côté de l'orifice central de la plaque.

Une telle exécution des plaques du porte-broche du centre d'appui assure un contrôle à vue du sens de placement de la rainure ouverte au moment de l'installation des broches dans cette dernière.

Il est avantageux de réaliser des trous débouchants dans les faces en bout des plaques du porte-broche et d'y placer des aimants.

Suivant un autre mode de réalisation de l'invention, on peut exécuter des trous débouchants dans les faces en bout des joints du porte-broche et y placer des aimants.

De telles variantes de l'invention assurent le maintien préalable et provisoire des broches dans un sens déterminé avant leur fixation complète.

Il est avantageux de réaliser des entailles sur la face cylindrique des plaques du porte-broche.

La réalisation de ces entailles simplifie l'orientation de la rainure ouverte des plaques du porte-broche.

On peut plier les branches des broches suivant un angle obtu.

Une telle forme des broches permet d'augmenter la rigidité du centre d'appui du porte-broche et de faciliter l'installation des broches avec croisement.

Suivant encore un autre mode de réalisation de l'invention, il est également possible de plier les branches des broches suivant un angle aigu.

Cela permet de poser les porte-broches sur les broches du côté extérieur de celles-ci, ce qui est particulièrement avantageux pour réduire au minimum les dimensions de l'appareil. De plus, on peut disposer la tige filetée le plus près possible de l'os, ce qui accroît la rigidité des broches et augmente les efforts transmis.

Il est avantageux de réaliser sur des butées les branches des broches qui entrent dans l'os pour limiter l'introduction de la broche dans l'os.

Ces butées sont nécessaires pour prévenir les lésions des tendons sur la face palmaire de la main en limitant la longueur de la broche introduite dans l'os, ainsi que pour accroître la rigidité de la fixation.

D'autre part l'appareil selon l'invention comporte deux centres d'appui et peut avoir un centre d'appui complémentaire, installé sur une tige filetée complémentaire pouvant se déplacer par rapport à la tige filetée principale.

L'introduction dans l'appareil d'un centre d'appui complémentaire permet de réaliser le traitement simultané d'os adjacents ou voisins.

Suivant une variante de réalisation de l'invention, le centre d'appui complémentaire est installé avec possibilité de déplacement relatif sur une tige filetée complémentaire dont l'axe longitudinal est essentiellement parallèle à l'axe longitudinal de la tige filetée principale et qui est installée avec possibilité de déplacement relatif le long de son axe.

Cela permet d'allonger l'os tubulaire court le plus proche qui s'articule avec le fragment du moignon de la phalange.

Suivant encore une autre variante de réalisation de l'appareil selon l'invention, ce dernier comporte une plaque avec des trous dans lesquels se placent les tiges filetées principale et complémentaire qui sont reliées à la plaque au moyen d'écrous qui sont disposés des deux côtés de la plaque.

Une telle réalisation de l'invention permet d'effectuer une extension pour allonger l'os tubulaire court situé à proximité grâce à la tige filetée principale ou la tige complémentaire.

Suivant encore une autre variante de réalisation de l'appareil selon l'invention, le centre d'appui complémentaire se place sur une tige filetée complémentaire qui est reliée à la tige filetée principale avec possibilité de déplacement angulaire relatif.

Une telle construction rend possible, simultanément à l'élongation de l'os tubulaire court, l'écartement des os adjacents, par exemple pour élargir les espaces interdigitaux.

Dans une variante suivante de l'invention, la tige filetée complémentaire est articulée avec l'un des bouts d'une seconde tige filetée complémentaire, dont le second bout est installé avec possibilité de déplacement longitudinal ainsi que de fixation au moyen d'écrous dans un support qui se situe sur la tige filetée principale et entre les deux centres d'appui.

Cela permet d'élargir les espaces interdigitaux et en même temps d'élonger l'un des os tubulaires courts. Dans ce cas les axes longitudinaux des tiges filetées principale et complémentaire se placent dans le sens des rayons des doigts.

Selon encore une autre variante de réalisation de l'appareil selon l'invention, ce dernier est muni d'un bloc pour écarter les parties molles, qui comporte une broche recourbée en forme de boucle dont les branches se fixent dans un porte-broche et passent à travers ce dernier en se croisant, dans ce cas, au moins l'une des branches se fixe sur l'un des bouts de la première tige filetée complémentaire dont le second bout est installé avec possibilité de déplacement longitudinal, l'un des bouts d'une seconde tige filetée complémentaire dont le second bout est installé avec possibilité de déplacement longitudinal dans une seconde plaque à trous, ladite plaque étant elle-même installée sur la tige filetée principale avec possibilité de déplacement relatif le long de son axe longitudinal, la tige filetée principale et les tiges filetées complémentaires étant situées parallèlement, alors que le prolongement de l'axe longitudinal de la première tige filetée complémentaire se confond à l'axe de symétrie de la boucle de broche et se situe au niveau du croisement des broches des centres d'appui.

Une telle réalisation de l'invention avec introduction des broches dans les sites optimaux de l'os permet de créer une réserve de parties molles à l'extrémité distale de l'os à une cadence plus élevée que la cadence d'allongement de l'os, sans toutefois produire d'altérations trophiques dans la phalange terminale.

Par ailleurs, dans certains cas le dispositif pour ostéosynthèse comportant deux appareils selon l'invention peut comporter une tige filetée d'extension qui est installée avec possibilité de déplacement longitudinal ainsi que de fixation au moyen d'écrous dans des supports et qui sont articulées avec les bouts correspondants des tiges filetées principales.

Cela offre la possibilité d'allonger simultanément les deux os métacarpaux adjacents et d'élargir les espaces interdigitaux des os soumis à l'élongation. Dans ce cas, les axes longitudinaux des tiges filetées principales sont situés parallèlement aux rayons des os de la main.

Brève description des dessins

L'invention sera mieux comprise à l'aide des exemples de réalisation qui suivent, et se réfèrent aux dessins schématiques annexés, dans lesquels :

La figure 1 représente l'appareil de compression-extension pour ostéosynthèse selon l'invention, posé sur les fragments d'os tubulaire court ;

La figure 2 représente le centre d'appui de l'appareil selon l'invention, à son état détaché ;

La figure 3 représente la plaque du porte-broche en forme de rondelle ;

La figure 4 représente le joint du porte-broche en forme de rondelle ;

La figure 5 représente une variante de fixation des broches dans le centre d'appui de l'appareil selon l'invention, les branches des broches étant pliées suivant un angle obtu ;

La figure 6 représente une variante éventuelle de fixation des broches dans le centre d'appui de l'appareil selon l'invention, les branches des broches étant pliées suivant un angle aigu ;

La figure 7 représente l'appareil selon l'invention, posé sur une mandibule présentant une lésion ;

La figure 8 représente l'appareil selon l'invention avec trois centres d'appui, posé sur un os tubulaire long présentant une double fracture ;

La figure 9 représente une variante de l'appareil selon l'invention avec un centre d'appui complémentaire apte à effectuer un déplacement alternatif ;

La figure 10 représente une variante de l'appareil selon l'invention avec un centre d'appui complémentaire apte à effectuer un déplacement angulaire ;

La figure 11 représente une variante de l'appareil selon l'invention avec un bloc d'écartement des parties molles ;

La figure 12 représente une variante de dispositif composé de deux appareils selon l'invention.

Mode de réalisation préféré de l'invention

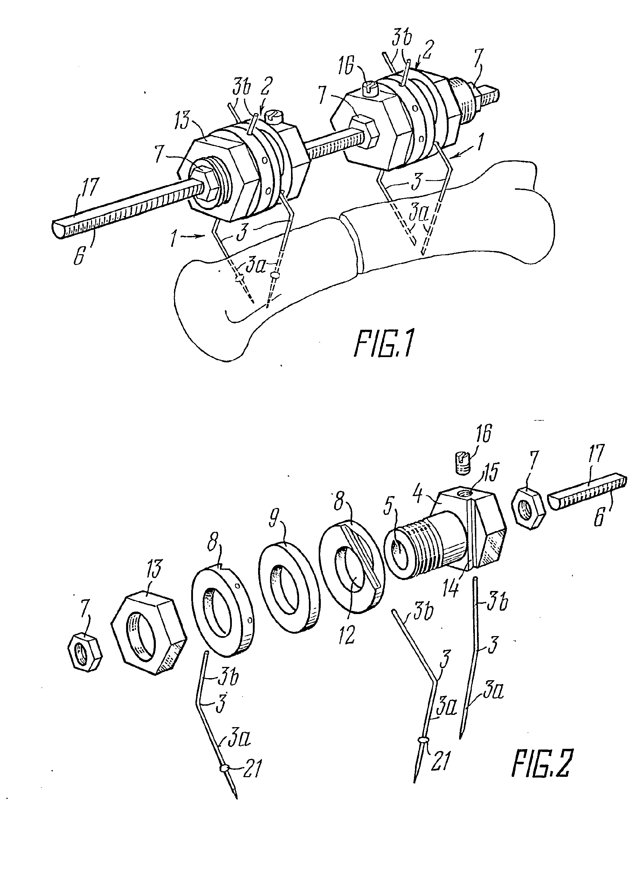

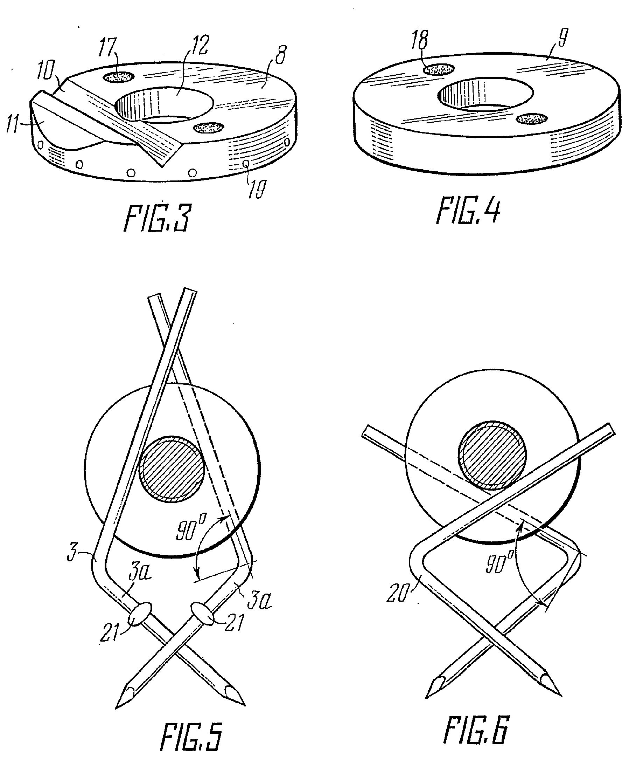

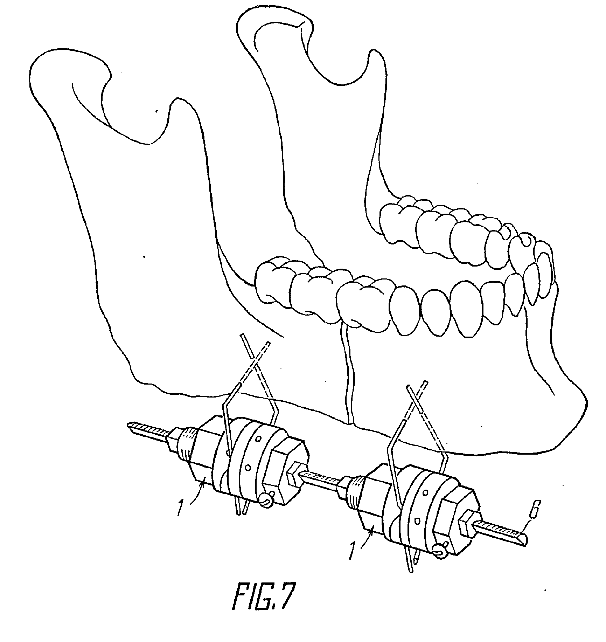

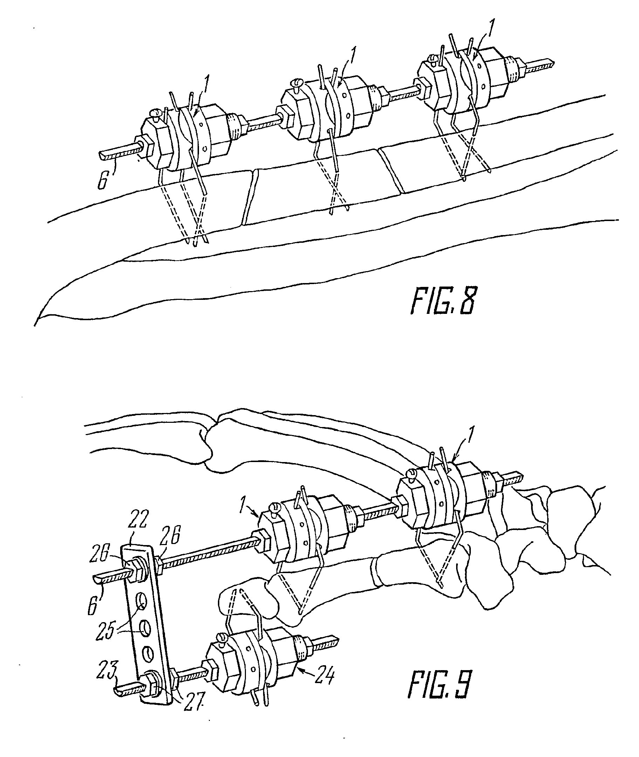

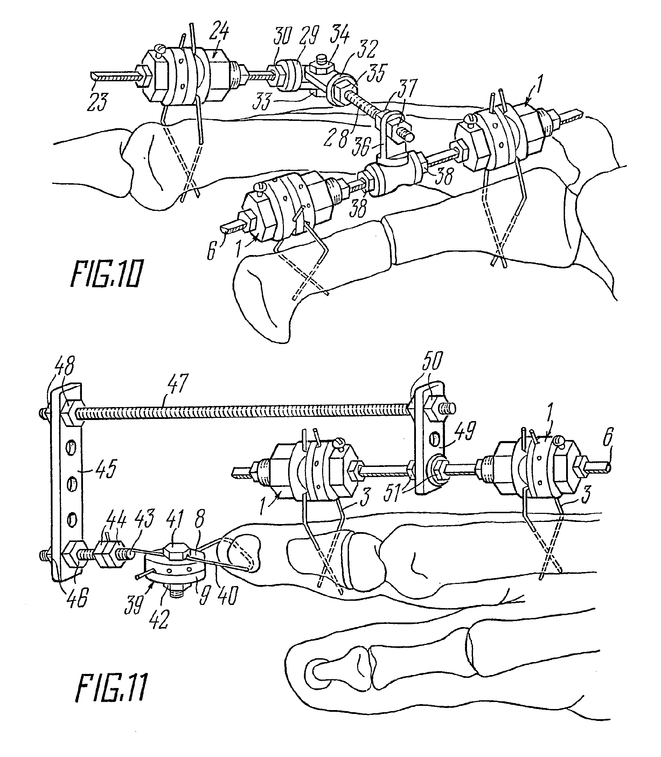

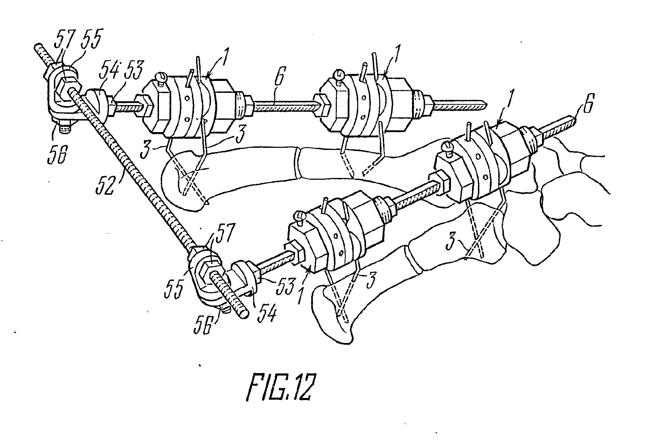

En se reportant à la figure 1 qui représente l'appareil de compression-extension pour ostéosynthèse selon l'invention en vue générale et à la figure 2 qui représente le centre d'appui en état désassemblé, on voit que l'appareil selon l'invention comporte au moins deux centres d'appui 1. Chaque centre d'appui 1 comporte un porte-broche 2 avec des broches 3 y fixées. Des branches 3a des broches 3 se croisent dans l'os, alors que des branches 3b des broches 3 sont fixées en se croisant dans les porte-broches 2. Chacun des porte-broches 2 est réalisé en forme de boulon 4 avec un orifice axial 5 et est installé avec possibilité de déplacement longitudinal le long d'une tige filetée principale 6. Le déplacement s'effectue au moyen d'écrous 7 qui sont installés de part et d'autre du boulon 4 du porte-broche 2. Sur le boulon 4 du porte-broche 2 sont disposées des plaques 8 et des joints 9 avec possibilité de rotation. Les plaques 8 (figure 3) et les joints 9 (figure 4) sont réalisés en forme de rondelles. Les faces en bout des plaques 8 portent des rainures ouvertes 10. La plaque 8 du porte-broche 2 comporte un méplat 11 dont le plan est incliné par rapport à la face en bout de la plaque 8 est est parallèle à l'axe longitudinal de la rainure ouverte 10. Le méplat 11 et la rainure ouverte 10 sont réalisés du même côté d'un orifice central 12 de la plaque 8. Les broches 3 sont fixées entre les faces en bout des plaques 8, des joints 9 et de la tête du boulon 4 par un écrou de serrage 13 (figures 1, 2). La face en bout de la tête du boulon 4 comporte une rainure ouverte 14 pour l'installation dans le porte-broche 2 de la troisième broche 3.

Par ailleurs, la tête du boulon 4 comporte, perpendiculairement à l'axe de l'orifice 5, un trou fileté 15 dans lequel se place une vis d'arrêt 16. L'extrémité de la vis d'arrêt 16 coopère avec un méplat 17 réalisé le long de la tige filetée principale 6.

Suivant les variantes de réalisation de l'appareil selon l'invention, les faces de bout des plaques 8 (figure 3) et/ou des joints 9 (figure 4) peuvent comporter des aimants 17, 18 respectivement dans des trous débouchants. La surface cylindrique des plaques 8 peut comporter des entailles 19.

La figure 5 représente une variante d'exécution de l'invention dans laquelle les broches 3 sont repliées suivant un angle obtu, et la figure 6 montre la variante suivant laquelle les broches 20 sont repliées suivant un angle aigu.

Pour limiter la profondeur d'introduction des broches 3 dans l'os, les branches 3a peuvent comporter des butées 21.

L'appareil de compression-extension pour ostéosynthèse selon l'invention peut, en fonction des tâches thérapeutiques à effectuer, être muni d'une quantité adéquate de centres d'appui.

Pour la fixation des os courts et, par exemple de la mandibule, on utilise un appareil selon l'invention qui est muni de deux centres d'appui 1 (figure 7).

Pour la fixation des os tubulaires longs on utilise trois centres d'appui 1 (figure 8) installés sur une tige filetée principale 6.

Pour l'élongation d'un os court tubulaire articulé à proximité du fragment de moignon de phalange, l'appareil selon l'invention est muni de deux centres d'appui 1 (figure 9) installés sur la tige filetée principale 6 qui se réunit par le biais d'une plaque 22 avec une tige filetée complémentaire 23 qui porte un centre d'appui complémentaire 24. La plaque 22 porte des trous 25 par lesquels passent la tige filetée principale 6 et la tige complémentaire 23, qui sont toutes deux fixées dans ladite plaque au moyen d'écrous 26 et 27, respectivement.

Pour l'élargissement des espaces interdigitaux et en même temps l'élongation de l'un des os courts tubulaires, l'appareil selon l'invention représenté à la figure 10 comporte deux centres d'appui 1, installés sur la tige filetée principale 6, alors que le centre d'appui complémentaire 24 est installé sur la tige filetée complémentaire 23.

La tige filetée complémentaire 23 est reliée à une seconde tige filetée complémentaire 28. L'une des extrémités de la tige filetée complémentaire 23 est reliée à un support 29 au moyen d'un écrou 30. Le support 29 est relié à un support 32 au moyen d'un boulon 33 et d'un écrou 34 avec possibilité de déplacement angulaire. Le support 32 est relié à l'une des extrémités de la seconde tige filetée complémentaire 28 à l'aide d'un écrou 35. L'autre extrémité de la seconde tige filetée complémentaire 28 est disposée dans un support 36 avec possibilité de déplacement longitudinal au moyen d'écrous 37. Le support 36 se place sur la tige filetée principale 6 entre les deux centres d'appui 1 avec possibilité de déplacement longitudinal à l'aide d'écrous 38.

L'appareil selon l'invention représenté sur la figure 11 comporte deux centres d'appui 1 qui sont interreliés par le biais de la tige filetée principale 6, et un bloc 39 pour l'écartement des parties molles. Le bloc 39 pour l'écartement des parties molles comporte une broche 40 courbée en forme de boucle. Les branches de la broche 40 sont fixées en croix entre les faces en bout de la plaque 8, du joint 9 et de la tête du boulon 41 au moyen d'un écrou 42. L'une des branches de la

- 16 -

broche 40 est fixée sur l'une des extrémités de la première tige filetée complémentaire 43 au moyen d'écrous 44. L'autre extrémité de la première tige filetée complémentaire 43 est installée avec possibilité de déplacement longitudinal dans la première plaque 45 à l'aide des trous et y est fixé par des écrous 46. La plaque 45 comporte également avec possibilité de déplacement longitudinal, l'une des extrémités de la seconde tige filetée complémentaire 47 où celle-ci est fixée par des écrous 48. L'autre extrémité de la seconde tige filetée complémentaire 47 est installée dans le trou de la seconde plaque 49 où elle est fixée à l'aide d'écrous 50 permettant d'effectuer des déplacements longitudinaux. La seconde plaque 49 se place au moyen d'un orifice sur la tige filetée principale 6 entre les centres d'appui. Le déplacement de la seconde plaque 49 le long de la tige filetée principale 6 est réalisé grâce à des écrous 51. La tige filetée principale 6 et les deux tiges filetées complémentaires 43 et 47 sont disposées parallèlement. En outre, l'axe de symétrie de la boucle de la broche 40 est situé au niveau du croisement des broches 3 et des centres d'appui 1.

Le dispositif suivant l'une des variantes de réalisation de l'invention qui est représenté sur la figure 12 comporte deux appareils selon l'invention qui sont réunis entre eux par une tige filetée d'extension 52. Les extrémités de même sens des tiges filetées principales 6, comportant chacune deux centres d'appui 1, sont reliées à des supports 54 à l'aide d'écrous 53. Les supports 54 sont eux-mêmes reliés à des supports 55 au moyen d'écrous 56. Les extrémités de la tige filetée d'extension 52 sont placées dans les trous des supports 55 et y sont fixées par des écrous 57. Le déplacement longitudinal des supports 55 le long de la tige filetée de distraction est effectué au moyen d'écrous 57.

L'appareil de compression-extension pour ostéosynthèse est placé de la façon suivante.

On introduit dans l'os au moins deux paires de broches 3 suivant une angle déterminé l'une par rapport à l'autre. Les paires de broches 3 se placent distalement et à proximité. On introduit alors les broches suivant un angle déterminé l'une par rapport à l'autre de façon que le croisement de chacune des paires de branches 3a des broches distales et la plus proche 3 pénétrent dans l'os sur la profondeur de la couche corticale opposée.

Par ailleurs, avant l'intervention il convient de préparer un nombre requis de porte-broches 2 des centres d'appui 1. Lors de la préparation des porte-broches on installe sur les boulons 4 les orifices axiaux 5 des lots de plaques 8 avec des rainures ouvertes 10 et des joints 9, ainsi que des écrous de serrage 13.

On replie les branches 3b des paires de broches les plus proches 3 et on les place à tour de rôle de façon qu'elles se croisent dans les rainures ouvertes 10 des plaques 8 des porte-broches 2 suite à quoi on les fixe avec l'écrou de serrage 13. On installe ensuite l'un des porte-broches 2 sur la tige filetée principale 6, on l'oriente à l'aide de la vis d'arrêt 16 et on le fixe avec les écrous 7. On installe alors le second porte-broche 2 et on serre la vis d'arrêt 16. On replie les branches des paires de broches distales 3 et on les place à tour de rôle dans les rainures ouvertes 10 des plaques 8 du second porte-broche 2, installé entre les écrous 7. On fixe ensuite les branches 3b des broches distales 3 au moyen de l'écrou de serrage 13 puis on place l'axe de la tige filetée principale 6 de manière aussi parallèle que possible à l'axe longitudinal de l'os.

Dans les cas où il s'avère nécessaire d'installer des broches supplémentaires ou d'installer et de fixer les broches 3 dans le centre d'appui 1 avec un nombre de plaques 8 et de joints 9 le plus réduit possible, on utilise la rainure ouverte 14 située sur la face en bout de la tête du boulon 4.

Pour simplifier l'installation et vérifier visuellement au cours de la mise en place des branches 3b des broches 3 la direction de la rainure ouverte sur les faces en bout des plaques 8 du porte-broche 2, on utilise le méplat 11 dont le plan est parallèle à l'axe de la rainure 10. A cette fin on fait tourner la plaque 8 autour du boulon 4 à l'aide d'un objet pointu par exemple d'une aiguille ou d'une broche que l'on introduit dans l'entaille correspondante 19. En outre, pour le maintien provisoire de l'une des branches 3b de la broche 3 installée dans la rainure ouverte 10, on utilise les aimants 17 et 18 au moyen desquels les plaques 8 et les joints 9 sont attirées l'un vers l'autre et obturent ainsi la rainure 10 en fixant la broche 3.

Après la mise en place complète de l'appareil sur l'os, on desserre les vis d'arrêt 16 puis on dévisse les écrous 7 et on retire la tige filetée principale 6. Ensuite, on réalise par les incisions de la peau l'ostéotomie et on remet en place l'appareil.

L'extension s'effectue par déplacement des paires d'écrous 7 sur la tige filetée principale 6. On déplace les paires d'écrous 7 des porte-broches distal et le plus proche 2 en sens mutuellement opposés.

La rotation du centre d'appui 1 au cours du déplacement dosé de la tige filetée principale 6 le long de l'axe évite l'intéraction de la vis d'arrêt 16 avec le méplat 17 de la tige filetée principale 6.

Quand il est nécessaire de fixer le fragment du moignon de la phalange et d'allonger l'os tubulaire court qui s'articule à proximité dudit fragment du moignon de la phalange, on utilise un centre d'appui 24 complémentaire que l'on installe sur une tige filetée complémentaire 23 montrée sur la figure 9. On réunit alors la tige filetée complémentaire .23 et la tige filetée principale 6 par l'intermédiaire de la plaque 22 et des écrous 23 et 26. La tige filetée principale 6, la tige filetée complémentaire 23 et l'axe de l'os que l'on allonge doivent être parfaitement parallèles.

L'extension du fragment de moignon phalangien se réalise à l'aide des écrous 23 à une cadence supérieure à l'extension du fragment ostéotomisé du moignon qui est réalisée par les écrous 26.

Dans le cas où il est nécessaire d'élargir les espaces interdigitaux et en même temps que d'allonger l'un des os tubulaires courts, on a recours à un centre d'appui complémentaire 24 dans lequel on installe des broches 3, on le fixe de façon immobile sur la tige filetée complémentaire 23 que l'on réunit au support 29 au moyen de l'écrou 30. Le support 29 est relié au support 32 au moyen du boulon 33 et de l'écrou 34 et est relié à la tige filetée complémentaire 28 au moyen de l'écrou 35. On réunit alors la seconde tige filetée complémentaire 28 et la tige filetée principale 6 par l'intermédiaire du support 36.

On effectue le déplacement angulaire choisi par déplacement des écrous 37 le long de la seconde tige filetée complémentaire 28.

L'une des variantes éventuelles de l'appareil prévoit son montage avec le bloc prévu pour écartement des parties molles représenté dans la figure 11. L'écartement des parties molles s'effectue à l'aide du bloc dans lequel on fait passer la broche 40 repliée en forme de boucle à travers le fragment osseux (qui n'est pas représenté dans la figure) suite à quoi on serre celle-ci au moyen de la plaque 8, du joint 9, du boulon 41 et de l'écrou 42. On réunit la branche de la broche avec la première tige filetée complémentaire 43 que l'on relie alors à la première plaque au moyen des écrous 46. On réunit la première plaque 45 avec la seconde tige filetée complémentaire 47 à l'aide des écrous 48. On réunit la seconde tige filetée complémentaire 47 avec la tige filetée principale 6 entre les centres d'appui 1 au moyen de la seconde plaque 43 et des écrous 50 et 51.

Toutes les tiges filetées 6, 43, 47 doivent être disposées parallèlement entre elles et par rapport à l'axe de l'os soumis à l'allongement.

L'extension est effectuée à l'aide des écrous 46 disposés le long de la tige 43 avec une cadence supérieure à celle de l'extension effectuée par la tige principale 6. On peut déplacer le bloc d'écartement des parties molles sur la seconde tige filetée complémentaire 47 avec les écrous 48, 50.

Dans le cas où il est nécessaire d'allonger simultanément deux os métacarpaux adjacents et d'élargir les espaces interdigitaux des os que l'on allonge, on a recours à un dispositif composé de deux appareils selon l'invention comme on le voit bien sur la figure 12.

Pour ce faire, on pose deux appareils selon l'invention sur les os métacarpaux adjacents et on réunit dans les supports 54 les deux extrémités de même sens des tiges filetées principales au moyen des écrous 53, puis on réunit les supports 54 avec les supports 55 à l'aide de l'écrou 56. On installe alors la tige filetée d'extension 52 dans les trous des supports 55 .

On réalise l'extension à l'aide d'écrous 7 des centres d'appui  1 disposés sur les tiges filetées principales 6, en effectuant simultanément l'extension à l'aide de la tige d'extension 52 des tiges filetées principales 6 au moyen des écrous 57, ce qui entraîne un déplacement angulaire des principales tiges filetées aboutissant à l'élargissement des espaces interdigitaux.

Application industrielle

L'appareil de compression-extension pour ostéosynthèse selon l'invention peut trouver son application dans le traitement des fractures des phalanges digitales ou des os métacarpaux. La pose de l'appareil de façon isolée sur chaque segment osseux du côté du dos de la main permet d'exclure au cours du traitement la contracture des articulations voisines et de réaliser avantageusement une éducation précoce de la main. Par ailleurs, l'appareil selon l'invention peut s'avérer efficace pour l'arthrodèse des articulations interphalangiennes et métacarpo-phalangiennes. Lors du traitement des défauts des os métacarpaux, l'appareil peut être posé non seulement sur 1 ou 5 os métacarpaux, mais aussi sur 2, 3, 4 os métacarpaux.  On utilise également l'appareil de compression-extension selon l'invention pour ostéosynthèse pour l'allongement des os tubulaires courts. La structure de l'appareil selon l'invention permet également d'utiliser celui-ci dans les anomalies de développement des mains afin d'allonger des moignons digitaux. D'autre part le montage en nombre requis de centres d'appui permet la fixation du fragment du moignon phalangien et l'allongement simultané de l'os court qui s'y articule au plus proche ainsi que l'allongement des os tubulaires courts en élargissant simultanément les espaces interdigitaux.

L'appareil peut aussi trouver son application dans le traitement des lésions et des racourcissements du mandibule et des os tubulaires longs. L'utilisation de l'appareil selon l'invention permet aux malades d'utiliser cet appareil eux-mêmes.

EP 0 377 744 A1

- 23 -

Revendications.

1. Appareil de compression-extension pour ostéosynthèse du type comportant au moins deux centres d'appui (1) qui comprennent au moins deux broches (3) dont certaines branches (3a) se croisent dans l'os alors que les autres (3b) sont fixées dans des porte-broches (2) réalisées en forme de boulon (4) et comportant un ensemble de plaques (8) munies de rainures ouvertes (10) pour l'installation des broches (3) ainsi qu'un ensemble de joints (9), lesdits centres d'appui sont installés sur une tige filetée (6) avec possibilité de déplacement le long de son axe ainsi que de fixation au moyen d'écrous (7), caractérisé en ce que le boulon (4) du porte-broche (2) comporte un orifice axial (5) par lequel passe la tige filetée principale (6), les plaques (8) et les joints (9) sont réalisés en forme de rondelles installées sur le boulon (4) avec possibilité de rotation autour de son axe et se fixent à l'aide d'un écrou de serrage (13), les rainures ouvertes (10) pour le positionnement des broches (3) étant réalisées sur les faces en bout des plaques (8) de manière à passer celles-ci le long de la corde de leur circonférence à distance de l'orifice (12) formé dans la rondelle, et les écrous (7) sont installés sur la tige filetée principale (6), sont disposés des deux côtés des extrémités du boulon (4), les broches (3) étant repliées et fixées en croix dans le porte-broche (2).

2. Appareil selon la revendication 1, caractérisé en ce que le porte-broche (2) du centre d'appui (1) comporte une vis d'arrêt (16) installée dans un trou fileté (15) réalisé dans la tête du boulon (4) du porte-broche (2) de façon perpendiculaire à l'axe du trou (5) de ce dernier, et que le long de la tige filetée principale (6) est formé un méplat (17) avec lequel l'extrémité de la vis d'arrêt (16) coopère.

3. Appareil selon la revendication 1, caractérisé en ce que la face en bout du boulon (4) du porte-broche (2) comporte une rainure (14) pour l'installation de la broche (3) située du côté du filet précité.

4. Appareil selon la revendication 1, caractérisé en ce que chaque plaque (8) du porte-broche (2) comporte une rainure ouverte (10), réalisée en forme de rondelle et comporte un méplat (11) dont le plan est incliné par rapport à la surface d'extrémité de la plaque (8) et est parallèle à l'axe longitudinal de la rainure (10), ledit méplat (11) et la rainure (10) étant exécutés du même côté de la plaque (8) par rapport à l'orifice (12).

5. Appareil selon la revendication 1, caractérisé en ce que les faces en bout des plaques (8) des porte-broches (2) comportent des trous débouchants dans lesquels sont disposés des aimants (17).

6. Appareil selon la revendication 1, caractérisé en ce que les faces en bout des joints (9) du porte-broche (2) comportent des trous débouchants dans lesquels sont disposés des aimants (18).

7. Appareil selon la revendication 1, caractérisé en ce que la surface cylindrique des plaques (8) du porte-broche (2) comporte des entailles (19).

8. Appareil selon la revendication 1, caractérisé en ce que les branches (3a, 3b) des broches (3) du centre d'appui (1) sont repliées suivant un angle obtu.

9. Appareil selon la revendication 1, caractérisé en ce que les branches des broches (20) du centre d'appui (1) sont repliées suivant un angle aigu.

10. Appareil selon la revendication 1, caractérisé en ce que les branches (3a) des broches (3) qui pénètrent dans l'os comportent des butées (21) pour limiter la profondeur d'introduction de la broche (3) dans l'os.

11. Appareil selon la revendication 1 du type comportant deux centres d'appui (1), caractérisé en ce qu'il comprend de plus un centre d'appui complémentaire (24) disposé sur une tige filetée complémentaire (23) avec possibilité de déplacement par rapport à la tige filetée principale (6).

12. Appareil selon la revendication 11, caractérisé en ce que le centre d'appui complémentaire (24) est disposé sur la tige filetée complémentaire (23) avec possibilité de déplacement relatif, l'axe longitudinal de ladite tige étant essentiellement parallèle à l'axe longitudinal de la tige filetée principale (6) et est installé avec possibilité de déplacement relatif le long de son axe.

13. Appareil selon la revendication 12, caractérisé en ce qu'il comporte une plaque (22) munie de trous (25) dans lesquels sont placées les tiges filetées principale et complémentaire (6, 23) et sont reliées à la plaque (22) au moyen d'écrous (26, 27) disposés des deux côtés de la plaque (22).

14. Appareil selon la revendication 11, caractérisé en ce que le centre d'appui complémentaire (24) est installé sur une tige filetée complémentaire (23), réunie avec la tige filetée principale (6) avec possibilité de déplacement angulaire relatif.

15. Appareil selon la revendication 14, caractérisé en ce que la tige filetée complémentaire (23) est articulée sur l'une des extrémités d'une seconde tige filetée complémentaire (28), dont la seconde extrémité est installée avec possibilité de déplacement longitudinal et est fixée à l'aide d'écrous (37) dans le support (36) situé sur la tige filetée principale (6) entre les deux centres d'appui (1).

16. Appareil selon la revendication 1 du type comportant deux centres d'appui (1), caractérisé en ce qu'il est muni d'un bloc (39) d'écartement des parties molles qui comprend une broche (40) repliée en forme de boucle et dont les branches se fixent dans le porte-broche et passent à travers ce dernier en se croisant, au moins l'une des branches étant fixée sur l'une des extrémités de la première tige filetée complémentaire (43), l'autre extrémité étant installée avec possibilité de déplacement longitudinal dans une première plaque (45) avec des trous, dans laquelle se place également, avec possibilité de déplacement longitudinal, une extrémité d'une seconde tige filetée complémentaire (47) dont l'autre extrémité est installée avec possibilité de déplacement longitudinal dans la seconde plaque (49) avec des trous et qui est elle-même disposée sur la tige filetée principale (6) avec possibilité de déplacement relatif le long de son axe longitudinal, la tige principale (6) et les tiges filetées complémentaires (43, 47) étant disposées parallèlement et le prolongement de l'axe longitudinal de la première tige filetée complémentaire (43) se comportant avec l'axe de symétrie de la boucle de la broche (40) de façon à reposer au niveau du croisement des broches (3) des centres d'appui (1).

17. Dispositif pour ostéosynthèse selon l'une quelconque des revendications de 1 à 10, du type comportant deux appareils posés sur des os voisins, caractérisé en ce qu'il comprend une tige filetée d'extension (52), disposée avec possibilité de déplacement longitudinal et fixée au moyen d'écrous (57) dans des supports (55) qui sont réunis par une charnière avec les extrémités des tiges filetées principales (6) de même sens.

FIG.1

FIG.2

FIG.3

FIG.4

FIG.5

FIG.6

FIG.7

EP 0 377 744 A1

FIG.8

FIG.9

FIG.10

FIG.11

FIG.12

# INTERNATIONAL SEARCH REPORT

## I. CLASSIFICATION OF SUBJECT MATTER (if several classification symbols apply, indicate all) 6

According to International Patent Classification (IPC) or to both National Classification and IPC

IPC$^4$   A61B 17/60

## II. FIELDS SEARCHED

### Minimum Documentation Searched 7

| Classification System | Classification Symbols |
|---|---|
| IPC$^4$ | A61B 17/56, 17/58, 17/60 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| A | SU, A1, 1161100 (G.S.Sushko) 15 June 1985 (15.06.85) see the claims, figure 4, (cited in the description) | 1 |
| A | GB, A, 2146533 (TERRY A. CLYBURN) 24 April 1985 (24.04.85) see the claims, figures 1,2 (cited in the description)<br><br>& FR, A1, 2551650, 15.03.85<br>JP, A, 60-77747, 02.05.85<br>BR, A, 8404487, 30.07.85<br>US, A, 4628919, 16.12.86 | 11-16 |
| A | GB, A, 2168255 (ORTHOFIX SRL) 18 June 1986 (18.06.86) see the claims, figure 1, (cited in the description)<br>& BE, A, 903869, 16.04.86<br>SE, A, 8505962, 19.06.86<br>DK, A, 586485, 19.06.86<br>US, A, 4621627, 11.11.86<br>DE, C3, 3543042, 11.06.87<br>AU, B, 564568, 13.08.87 | 1 |

./.

* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 16 February 1989 (16.02.89) | 3 April 1989 (03.04.89) |
| International Searching Authority<br><br>ISA/SU | Signature of Authorized Officer |

Form PCT/ISA/210 (second sheet) (January 1985)

| Category* | Citation of Document, with indication, where appropriate, of the relevant passages | Relevant to Claim No |
|---|---|---|
| A | FR, A1, 2559380 (ORTHOFIX SRL) 16 August 1985 (16.08.85) see the claims,figure 1<br>& DE, C2, 3504616,   19.06.87<br>  GB, B , 2154144,   07.01.87<br>  JP, B , 61-55977,   29.11.86<br>  US, A , 4604997 ,   12.08.86<br>  NO, A , 850540 . ,   14.08.85<br>  BE, A1, 901716 ,   12.08.85<br>  DK, A , 64485   ,   14.08.85<br>  AU, A1, 3859485 ,   22.08.85<br>  SE, A , 8500624 ,   14.08.85 | 1 |
| A | DE, A1, 3527342 (AESCULAP-WERKE AG VORMALS JETTER & SCHEERER) 12 February 1987 (12.02.87) see the claims,figure 1 | 1 |
| A | US, A, 2251209 (OTTO STADER) 29 July 1941 (29.07.41) see the claims,figures 1,5,6 | 1 |
| A | US, A, 2393694 (O.S. KIRSCHNER) 29 January 1946 (29.01.46) see the claims,figures 1,5 | 1 |